(19) Europäisches Patentamt
European Patent Office
Office européen des brevets

(11) EP 0 719 544 B1

(12) **FASCICULE DE BREVET EUROPEEN**

(45) Date de publication et mention de la délivrance du brevet: **09.04.1997 Bulletin 1997/15**

(51) Int. Cl.$^6$: **A61K 7/50**, A61K 7/48

(21) Numéro de dépôt: **95402071.5**

(22) Date de dépôt: **13.09.1995**

(54) **Utilisation d'une composition contenant un alcool et un composé phosphate comme démaquillant**

Verwendung einer Zusammensetzung enthaltend einen Alkohol und eine Phosphate-Verbindung als Abschminkmittel

Use of composition containing an alcohol and a phosphate-containing derivative as a make-up remover

(84) Etats contractants désignés:
**AT BE CH DE ES FR GB IT LI NL SE**

(30) Priorité: **07.10.1994 FR 9412007**

(43) Date de publication de la demande:
**03.07.1996 Bulletin 1996/27**

(73) Titulaire: **L'OREAL**
**75008 Paris (FR)**

(72) Inventeurs:
- **Bui-Bertrand, Lien**
  **F-91600 Savigny Sur Orge (FR)**
- **Carrel, Marie-Laure**
  **F-75007 Paris (FR)**

(74) Mandataire: **Miszputen, Laurent**
**L'OREAL**
**Département Propriété Industrielle**
**Centre Charles Zviak**
**90, rue du Général Roguet**
**92583 Clichy Cédex (FR)**

(56) Documents cités:
**EP-A- 0 501 714**
**EP-A- 0 566 049**
**GB-A- 2 035 362**
**GB-A- 2 055 119**
**GB-A- 2 155 017**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Rank Xerox (UK) Business Services
2.14.2/3.4

EP 0 719 544 B1

## Description

La présente invention se rapporte à l'utilisation en cosmétique d'une composition, exempte d'ester d'acide gras et de diester de polyéthylèneglycol, comprenant un alcool et un composé phosphaté pour démaquiller la peau et/ou les yeux et/ou les cils et/ou les sourcils.

L'invention se rapporte également à un procédé de démaquillage qui consiste essentiellement à appliquer la composition selon l'invention sur de la peau et/ou des yeux et/ou des cils et/ou des sourcils maquillés.

Les démaquillants classiquement utilisés à ce jour sont des compositions ayant une concentration élevée en matières grasses dont la fonction, une fois appliquées sur la peau, est de dissoudre les différents corps gras présents sur la peau et notamment ceux qui sont contenus dans les produits de maquillage, de manière à les éliminer.

Ainsi, il est par exemple connu, par le document JP-A-55-150402, d'utiliser des compositions démaquillantes contenant des esters dont le nombre total d'atomes de carbone varie de 17 à 36 avec une préférence pour des esters ayant un nombre de carbone compris dans la plage de 23 à 28. La quantité efficace de ces esters est d'au moins 20% en poids par rapport au poids total de la composition.

Toutefois, l'utilisation de telles compositions démaquillantes à teneur élevée en corps gras provoque, au moment de leur application, un désagrément ou inconfort qui se traduit par une sensation de lourdeur sur le visage ou de voile sur les yeux. L'application de ces compositions sur les yeux peut, par ailleurs, entraîner un gonflement des paupières.

La concentration élevée en corps gras pose également des problèmes d'odeur désagréable. Il est par conséquent nécessaire de masquer cette odeur par un parfumage intense, ce qui provoque d'autres problèmes de tolérance.

En outre, en raison de leur texture "lourde", ces démaquillants manquent de fraîcheur, sont difficiles à travailler, et leur rinçage n'est pas aisé.

En effet, après leur application sur la peau, telle que celle des paupières, un rinçage à l'aide d'un tonique ou d'eau s'avère indispensable.

On connait par ailleurs, par l'ouvrage de Charles ZVIAK "Science des traitements capillaires" que les tensioactifs cationiques sont de médiocres détergents et sont, en général, aggressifs vis-à-vis de la peau du visage. Aussi, l'utilisation de tels tensioactifs pour démaquiller la peau du visage et/ou des yeux peut provoquer des rougeurs sur la peau non seulement du fait de leur aggressivité, mais aussi du fait de leurs mauvaises qualités détergentes obligeant des passages répétés d'un coton à démaquiller imprégné de la composition les contenant.

Aussi, il subsiste le besoin d'une composition démaquillante qui ne nécessite pas de rinçage après utilisation sur la peau, qui présente une très faible concentration en corps gras, évitant ainsi l'effet de "lourdeur" sur la peau, et qui nettoie et/ou démaquille la peau sans aggresser le tissu cutané du fait de la nature des détergents, ou sans la nécessité de nombreux passages sur la peau d'un coton imprégné de la composition.

On connait dans l'art antérieur des composés quaternaires phosphatés cationiques dans des compositions détergentes et moussantes pour l'hygiène de la peau ou des cheveux. Ils sont décrits dans les demandes de brevet GB-A-2 035 362 et GB-A-2 055 119.

Selon la demande EP-A-0 501 714, ces mêmes composés quaternaires phosphatés cationiques ont été aussi utilisés en association avec des polysaccharides cationiques dans des émulsions pour la protection de la peau contre les radiations ultraviolettes en vue d'augmenter la rétention en eau après lavage à l'eau et d'augmenter la résistance au lavage de l'effet filtrant.

Selon la demande EP-A-0 566 049, ces mêmes composés quaternaires phosphatés cationiques ont été aussi utilisés en association avec des alcools polyhydriques comme agents émulsionnants dans des shampooings conditionneurs à base de tensio-actifs anioniques détergents et de silicones insolubles dans l'eau.

La demanderesse a découvert de façon surprenante l'utilisation d'une composition comprenant au moins un premier composé choisi parmi les alcools mono- ou polyfonctionnels et au moins un second composé choisi parmi les composés quaternaires phosphatés de formule (I)

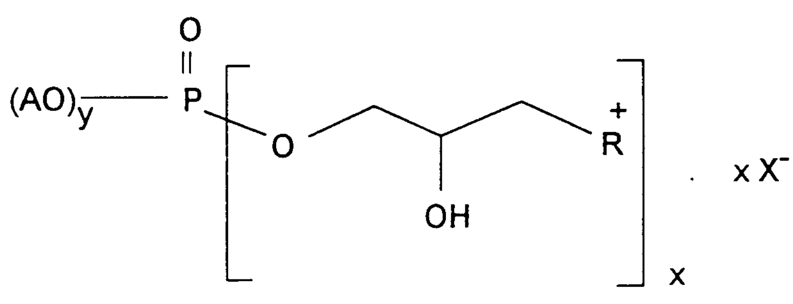

(I)

dans laquelle

- R est une amine tertiaire ayant au moins 6 atomes de carbone et qui peut être cyclique ou non cyclique, aliphatique, aromatique ou hétérocyclique,

- X est un anion,

- A est un métal alcalin,

- x et y sont deux nombres entiers tels que (x+y) = 3 , x ne pouvant être nul, la composition étant exempte d'ester d'acide gras et de diester de polyéthylèneglycol, pour démaquiller la peau et/ou les yeux et/ou les cils et/ou les sourcils sans aucune gène ou irritation et avec une excellente efficacité.

La présente invention présente l'avantage d'avoir des propriétés glissante et filmogène, ces propriétés permettant de ressentir ainsi une sensation de confort, tout en évitant le caractère aggressif et irritant des tensioactifs cationiques classiques vis-à-vis de la peau.

Un autre avantage procuré par la composition selon l'invention est de rendre les paupières douces lorsque ces dernières sont démaquillées par cette composition.

Par ailleurs, la composition selon l'invention permet de réaliser un démaquillage sans rinçage obligatoire, ce qui est particulièrement intéressant pour des peaux ayant certaines affections cutanées ou dans les cas de conditions peu propices à un rinçage de la peau, tel que les voyages.

La composition selon l'invention permet également son emploi dans des pays chauds, où l'utilisation de démaquillants trop riches en corps gras donne une sensation de lourdeur sur la peau, souvent désagréable à supporter.

Selon l'invention le radical R représente de préférence un motif amidoamine de formule (II) suivante :

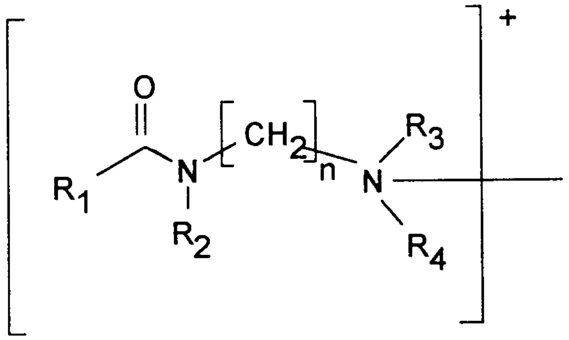

(II)

dans laquelle

- $R_1$ est choisi parmi un groupement alkyl, alcényl, alcoxy ou hydroxyalkyl ayant de 5 à 22 atomes de carbone chacun, ou un groupement aryl ou alkylaryl ayant jusqu'à 20 atomes de carbone,

- $R_2$ est choisi parmi l'atome d'hydrogène, un groupement alkyl, hydroxyalkyl ou alcényl ayant jusqu'à 6 atomes de carbone chacun, un groupement cycloalkyl ayant jusqu'à 6 atomes de carbone, et de préférence de 2 à 5 atomes de carbone, ou un groupement polyoxyalkylène ayant jusqu'à 10 atomes de carbone,

- $R_3$ et $R_4$, identiques ou différents, sont choisis parmi un groupement alkyl, hydroxyalkyl ou carboxyalkyl ayant jusqu'à 6 atomes de carbone dans chaque entité alkyl, ou un groupement polyoxyalkylène ayant jusqu'à 10 atomes de carbone, lesdits radicaux $R_3$ et $R_4$ pouvant par ailleurs former ensemble un N-hétérocycle avec l'atome d'azote auquel ils sont rattachés,

- n est un entier allant de 2 à 10.

L'anion entrant dans la formule (I) est de préférence un halogénure, tel qu'un ion bromure, iodure, fluorure ou mieux chlorure.

Le métal alcalin utilisé est, de préférence choisi parmi le sodium, le lithium et le potassium.

Selon un mode préféré de réalisation de l'invention, le second composé est choisi parmi le chloro- phosphate de stéaramidopropyl oxyéthyléné diammonium, le chloro-phosphate de linoléamidopropyl oxyéthyléné diammonium, le chloro- phosphate de cocamidopropyl oxyéthyléné diammonium, le chloro- phosphate de borageamidopropyl oxyéthyléné diammonium.

Ce second composé est généralement compris en une quantité allant de 0,5 à 3% en poids, et de préférence en une quantité allant de 1 à 2,5 % en poids par rapport au poids total de la composition.

Le premier composé est de préférence choisi parmi les alcools inférieurs ayant un nombre de carbone inférieur ou égal à 6, les polyéthylènes glycols (PEG) ayant un nombre de carbone inférieur ou égal à 50, éventuellement oxyéthyléné, et parmi leurs mélanges.

Le premier composé est de préférence choisi parmi le propylèneglycol, la glycérine, le sorbitol, le dipropylèneglycol, le butylèneglycol, l'hexylèneglycol, le mannitol et leurs mélanges.

De façon préférée, le premier composé est choisi parmi les PEG ayant de 6 à 14 motifs oxyéthylènes et plus préférentiellement parmi le PEG 8, le PEG 12. Le chiffre mentionné après PEG indique le nombre de motifs oxyéthylènes.

Ce premier composé est en général compris en une quantité inférieure ou égale à 20% en poids, et plus particulièrement en une quantité allant de 0,5 à 15% en poids par rapport au poids total de la composition.

Lorsque le premier composé est un alcool inférieur, il est de préférence présent en une quantité allant de 5 à 15 % en poids par rapport au poids total de la composition.

Lorsqu'il est choisi parmi les polyéthylène glycols, il est de préférence présent en une quantité inférieure ou égale à 15% en poids et par exemple en une quantité allant de 5 à 15% en poids et mieux de 0,5 à 3% en poids par rapport au poids total de la composition.

La composition selon l'invention peut aussi comprendre de plus un corps gras ne contenant pas de fonction ester. Ce corps gras peut être présent en une quantité au plus égale a 20% en poids par rapport au poids total de la composition et de préférence en une quantité allant de 0,05 à 10 % en poids.

Parmi les corps gras ne contenant pas de fonction ester, on peut citer les cires telles que la cire de silicone, les huiles synthétiques telles que la vaseline, les isoparaffines hydrogénées, l'isohexadécane.

La composition selon l'invention peut également comprendre de plus au moins un additif ne comportant pas de fonction ester choisi parmi les conservateurs, les tampons, les parfums, les actifs hydrophiles, les agents complexants, les gélifiants, et leurs mélanges.

Parmi les actifs, l'invention peut comprendre l'allantoïne, le bisabolol, l'acide glycyrrhétinique et ses sels, des vitamines, des huiles essentielles, des eaux florales telles que l'eau de tilleul stabilisée, un tampon.

La composition selon l'invention peut se présenter sous forme d'une émulsion (eau-dans-huile ou huile-dans-eau), d'une dispersion, d'un gel, d'une crème, d'une mousse, d'une lotion ou sous toute autre forme généralement utilisée dans le domaine cosmétique.

L'invention se rapporte également à un procédé de démaquillage qui consiste à appliquer sur de la peau et/ou des yeux et/ou des cils et/ou des sourcils maquillés une composition telle que décrite précédemment.

Elle se rapporte aussi à un procédé de nettoyage qui consiste à appliquer sur de la peau et/ou des yeux et/ou des cils et/ou des sourcils maquillés une composition telle que définie ci-dessus.

Ces différents procédés peuvent comprendre une étape de rinçage non obligatoire.

D'autres caractéristiques, aspects et avantages de la composition selon l'invention apparaîtront dans les exemples qui suivent, qui sont donnés à titre purement illustratif et nullement limitatif. Les proportions sont données en pourcentage en poids par rapport au poids total de la composition.

**EXEMPLE 1 :**

On a préparé ici une composition utilisable pour le démaquillage des paupières et des cils et/ou leur nettoyage, et qui se présente sous forme d'une lotion aqueuse.

| | |
|---|---|
| -Polyéthylène glycol 8 OE (premier composé) | 0,05 |
| -Chloro-phosphate de cocamidopropyle oxyéthyléné di-ammonium (second composé) | 2,13 |
| -Allantoïne (apaisant) | 0,05 |
| -Eau de tilleul stabilisé (apaisant) | 1 |
| -Conservateurs | 0,2 |
| -Tampons pour un pH = 7 | 1,3 |
| -Agent complexant (EDTA) | 0,1 |
| -Eau qsp | 100 |

**EXEMPLE 2:**

La composition selon cet exemple se présente sous forme d'une lotion utilisable pour le démaquillage et/ou le nettoyage de la peau et/ou du visage.

| *Phase grasse* : | |
|---|---|
| -Cire de silicone | 5 |

| *Phase aqueuse* : | |
|---|---|
| -Polyéthylène glycol 12 OE (premier composé) | 2 |
| -Chloro-phosphate de stéaramidopropyl oxyéthyléné di-ammonium (second composé) | 3 |
| - D-panthénol | 0,5 |
| - acide glycyrrhétinique(apaisant) | 1 |
| -Conservateurs | 0,2 |
| -Tampon pH =7 | 1,3 |
| -Eau qsp | 100 |

## Revendications

1. Utilisation en cosmétique d'une composition comprenant au moins un premier composé choisi parmi les alcools mono- ou polyfonctionnels et au moins un second composé choisi parmi les composés quaternaires phosphatés de formule (I)

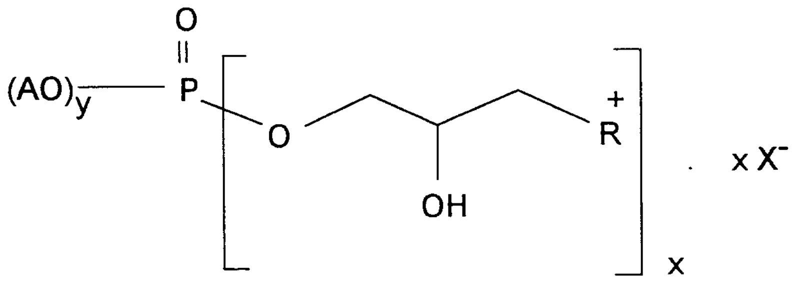

*(I)*

dans laquelle

- R est une amine tertiaire ayant au moins 6 atomes de carbone et qui peut être cyclique ou non cyclique, aliphatique, aromatique ou hétérocyclique,
- X est un anion,
- A est un métal alcalin,
- x et y sont deux nombres entiers tels que (x+y) = 3 , x ne pouvant être nul, la composition étant exempte d'ester d'acide gras et de diester de polyéthylèneglycol, pour démaquiller la peau et /ou les yeux et/ou les cils et/ou les sourcils.

2. Utilisation selon la revendication 1, caractérisée en ce que le radical R de la formule (I) est un motif amidoamine de formule (II) :

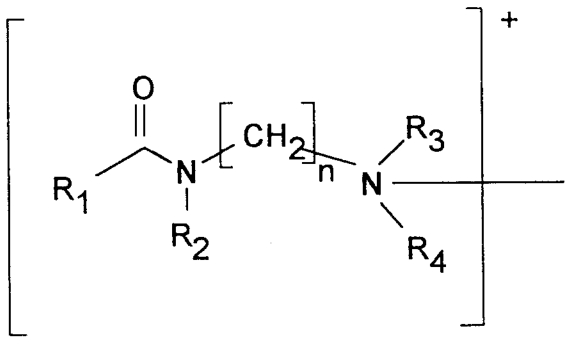

*(II)*

dans laquelle

- $R_1$ est choisi parmi un groupement alkyl, alcényl, alcoxy ou hydroxyalkyl ayant de 5 à 22 atomes de carbone chacun, ou un groupement aryl ou alkylaryl ayant jusqu'à 20 atomes de carbone,
- $R_2$ est choisi parmi l'atome d'hydrogène, un groupement alkyl, hydroxyalkyl ou alcényl ayant jusqu'à 6 atomes de carbone chacun, un groupement cycloalkyl ayant jusqu'à 6 atomes de carbone, et de préférence de 2 à 5 atomes de carbone, ou un groupement polyoxyalkylène ayant jusqu'à 10 atomes de carbone,
- $R_3$ et $R_4$, identiques ou différents, sont choisis parmi un groupement alkyl, hydroxyalkyl ou carboxyalkyl ayant jusqu'à 6 atomes de carbone dans chaque entité alkyl, ou un groupement polyoxyalkylène ayant jusqu'à 10 atomes de carbone, lesdits radicaux $R_3$ et $R_4$ pouvant par ailleurs former ensemble un N-hétérocycle avec l'atome d'azote auquel ils sont rattachés,
- n est un entier allant de 2 à 10.

3. Utilisation selon l'une des revendications 1 ou 2, caractérisée en ce que l'anion est choisi parmi les halogénures.

4. Utilisation selon l'une des revendications précédentes, caractérisée en ce que l'anion est un chlorure.

5. Utilisation selon l'une des revendications 1 à 4, caractérisée en ce que le second composé est choisi parmi le chloro- phosphate de stéaramidopropyl oxyéthyléné diammonium, le chloro- phosphate de linoléamidopropyl oxyéthyléné diammonium, le chlorophosphate de cocamidopropyl oxyéthyléné diammonium, le chloro- phosphate de borageamidopropyl oxyéthyléné diammonium.

6. Utilisation selon l'une des revendications précédentes, caractérisée en ce que le premier composé est choisi parmi les alcools inférieurs ayant un nombre de carbone inférieur ou égal à 6 et les polyéthylène glycols ayant un nombre de carbone inférieur ou égal à 50, éventuellement oxyéthylénés, et leurs mélanges.

7. Utilisation selon l'une des revendications précédentes, caractérisée en ce que le premier composé est choisi parmi le propylène glycol, la glycérine, le sorbitol, le dipropylène glycol, le butylène glycol, l'hexylène glycol, le mannitol et leurs mélanges.

8. Utilisation selon l'une des revendications 1 à 7, caractérisée en ce que le premier composé est choisi parmi les PEG ayant de 6 à 14 motifs oxyéthylènes.

9. Utilisation selon l'une des revendications précédentes, caractérisée en ce que la composition comprend en plus au moins un corps gras ne contenant pas de fonction ester.

10. Utilisation selon la revendication 9, caractérisée en ce que le corps gras est choisi parmi les cires de silicone, les huiles synthétiques ne contenant pas de fonction ester.

11. Utilisation selon l'une des revendications 9 ou 10, caractérisée en ce que le corps gras ne contenant pas de fonction ester est présent en une quantité au plus égale à 20 % en poids par rapport au poids total de la composition.

**12.** Utilisation selon l'une des revendications précédentes, caractérisée en ce qu'elle comprend de plus au moins un additif ne contenant pas de fonction ester choisi parmi les conservateurs, les tampons, les parfums, les actifs hydrophiles, les agents complexants.

**13.** Utilisation selon l'une des revendications précédentes, caractérisée en ce que le premier composé est présent en une quantité au plus égale à 20 % en poids par rapport au poids total de la composition.

**14.** Utilisation selon l'une des revendications précédentes caractérisée en ce que le premier composé, étant un alcool inférieur, est présent en une quantité allant de 0,5 à 15 % en poids par rapport au poids total de la composition.

**15.** Utilisation selon l'une des revendications précédentes, caractérisée en ce que le premier composé, étant un polyéthylène glycol, est présent en une quantité inférieure ou égale à 15 % en poids par rapport au poids total de la composition.

**16.** Utilisation selon l'une des revendications précédentes, caractérisée en ce que le premier composé, étant un polyéthylène glycol, est présent en une quantité allant de 5 à 15 % en poids par rapport au poids total de la composition.

**17.** Utilisation selon l'une des revendications précédentes, caractérisée en ce que le second composé est présent en une quantité allant de 0,5 à 3 % en poids par rapport au poids total de la composition.

**18.** Utilisation selon l'une des revendications précédentes, caractérisée en ce que le second composé est présent en une quantité allant de 1 à 2,5 % en poids par rapport au poids total de la composition.

**19.** Procédé de démaquillage de la peau et/ou des yeux et/ou des cils et/ou des sourcils maquillés, caractérisé en ce que l'on applique sur de la peau et/ou des yeux et/ou des cils et/ou des sourcils maquillés une composition selon l'une quelconque des revendications 1 à 18 et éventuellement, que l'on effectue ensuite un rinçage.

## Claims

**1.** Use in cosmetics of a composition comprising at least one first compound chosen from mono- or polyfunctional alcohols and at least one second compound chosen from quaternary phosphate compounds of formula (I)

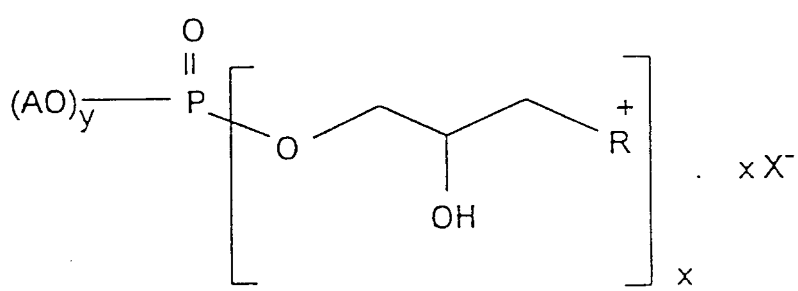

*(I)*

in which

- R is a tertiary amine having at least 6 carbon atoms and which can be cyclic or non-cyclic and aliphatic, aromatic or heterocyclic,
- X is an anion,
- A is an alkali metal,
- x and y are two integers such that $(x+y) = 3$ , it not being possible for x to be zero, the composition being free of fatty acid ester and of polyethylene glycol diester, for removing make-up from the skin and/or eyes and/or eyelashes and/or eyebrows.

**2.** Use according to Claim 1, characterized in that the R radical of the formula (I) is an amidoamine unit of formula (II):

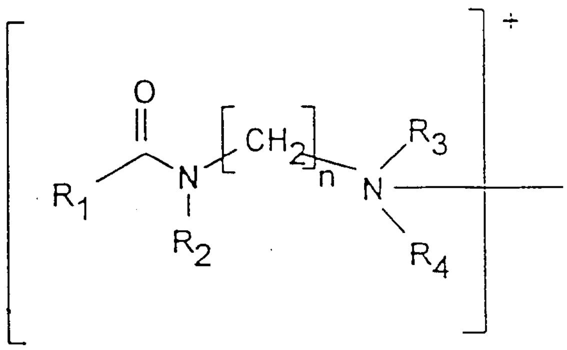

(II)

in which

- $R_1$ is chosen from an alkyl, alkenyl, alkoxy or hydroxyalkyl group, each having from 5 to 22 carbon atoms, or an aryl or alkylaryl group having up to 20 carbon atoms,
- $R_2$ is chosen from a hydrogen atom, an alkyl, hydroxyalkyl or alkenyl group, each having up to 6 carbon atoms, a cycloalkyl group having up to 6 carbon atoms, and preferably from 2 to 5 carbon atoms, or a polyoxyalkylene group having up to 10 carbon atoms,
- $R_3$ and $R_4$, which are identical or different, are chosen from an alkyl, hydroxyalkyl or carboxyalkyl group having up to 6 carbon atoms in each alkyl entity or a polyoxyalkylene group having up to 10 carbon atoms, it being possible for the said $R_3$ and $R_4$ radicals, moreover, together to form an N-heterocycle with the nitrogen atom to which they are attached,
- n is an integer ranging from 2 to 10.

**3.** Use according to either of Claims 1 or 2, characterized in that the anion is chosen from the halides.

**4.** Use according to one of the preceding claims, characterized in that the anion is a chloride.

**5.** Use according to one of Claims 1 to 4, characterized in that the second compound is chosen from stearamidopropyl oxyethylenated diammonium chlorophosphate, linoleamidopropyl oxyethylenated diammonium chlorophosphate, cocamidopropyl oxyethylenated diammonium chlorophosphate or borageamidopropyl oxyethylenated diammonium chlorophosphate.

**6.** Use according to one of the preceding claims, characterized in that the first compound is chosen from lower alcohols having a carbon number less than or equal to 6 and polyethylene glycols having a carbon number less than or equal to 50, which are optionally oxyethylenated, and their mixtures.

**7.** Use according to one of the preceding claims, characterized in that the first compound is chosen from propylene glycol, glycerol, sorbitol, dipropylene glycol, butylene glycol, hexylene glycol, mannitol and their mixtures.

**8.** Use according to one of Claims 1 to 7, characterized in that the first compound is chosen from PEGs having from 6 to 14 oxyethylene units.

**9.** Use according to one of the preceding claims, characterized in that the composition additionally comprises at least one fatty substance which does not contain an ester functional group.

**10.** Use according to Claim 9, characterized in that the fatty substance is chosen from silicone waxes or synthetic oils which do not contain an ester functional group.

**11.** Use according to either of Claims 9 or 10, characterized in that the fatty substance which does not contain an ester functional group is present in an amount not greater than 20 weight % with respect to the total weight of the composition.

**12.** Use according to one of the preceding claims, characterized in that it additionally comprises at least one additive which does not contain an ester functional group chosen from preservatives, buffers, fragrances, hydrophilic active agents or complexing agents.

13. Use according to one of the preceding claims, characterized in that the first compound is present in an amount not greater than 20 weight % with respect to the total weight of the composition.

14. Use according to one of the preceding claims, characterized in that the first compound, being a lower alcohol, is present in an amount ranging from 0.5 to 15 weight % with respect to the total weight of the composition.

15. Use according to one of the preceding claims, characterized in that the first compound, being a polyethylene glycol, is present in an amount less than or equal to 15 weight % with respect to the total weight of the composition.

16. Use according to one of the preceding claims, characterized in that the first compound, being a polyethylene glycol, is present in an amount ranging from 5 to 15 weight % with respect to the total weight of the composition.

17. Use according to one of the preceding claims, characterized in that the second compound is present in an amount ranging from 0.5 to 3 weight % with respect to the total weight of the composition.

18. Use according to one of the preceding claims, characterized in that the second compound is present in an amount ranging from 1 to 2.5 weight % with respect to the total weight of the composition.

19. Process for removing make-up from the skin and/or the eyes and/or the eyelashes and/or the eyebrows which have been made up, characterized in that a composition according to any one of Claims 1 to 18 is applied to skin and/or eyes and/or eyelashes and/or eyebrows which have been made up and, optionally, in that a rinsing is subsequently carried out.

**Patentansprüche**

1. Verwendung einer Zusammensetzung in der Kosmetik, die mindestens eine erste Verbindung, die unter den ein- oder mehrwertigen Alkoholen ausgewählt ist, und mindestens eine zweite Verbindung enthält, die unter den quaternären Phosphatverbindungen der Formel (I)

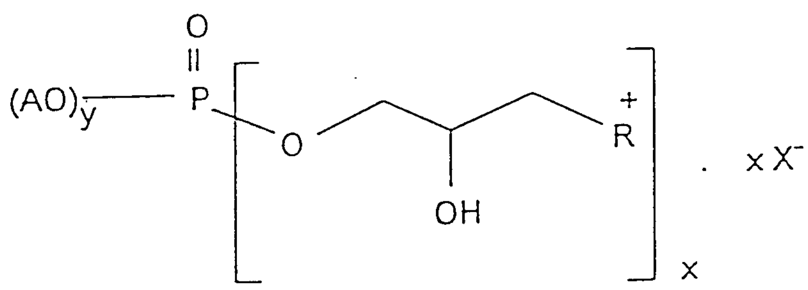 (I)

ausgewählt ist, worin bedeuten:

- R ein tertiäres Amin mit mindestens 6 Kohlenstoffatomen, das cyclisch oder nichtcyclisch, aliphatisch, aromatisch oder heterocyclisch sein kann,
- X ein Anion,
- A ein Alkalimetall,
- x und y ganze Zahlen, so daß gilt $(x+y) = 3$, wobei x nicht Null sein kann,

wobei die Zusammensetzung frei von Fettsäureestern und Polyethylenglykoldiestern ist, zum Abschminken der Haut und/oder der Augen und/oder der Wimpern und/oder der Augenbrauen.

2. Verwendung nach Anspruch 1, dadurch gekennzeichnet, daß die Gruppe R der Formel (I) eine Amidoamingruppe der Formel (II)

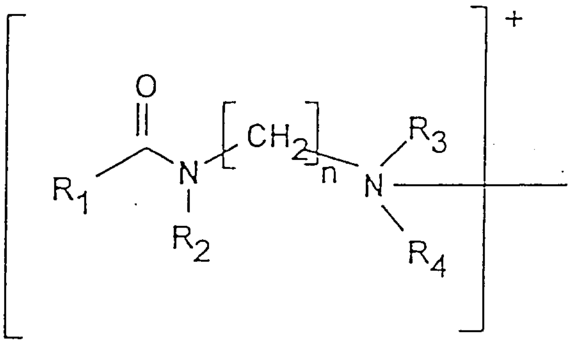

*(II)*

ist, worin bedeuten:

- $R_1$ Alkyl, Alkenyl, Alkoxy oder Hydroxyalkyl mit jeweils 5 bis 22 Kohlenstoffatomen oder Aryl oder Alkylaryl mit bis zu 20 Kohlenstoffatomen,
- $R_2$ Wasserstoff, Alkyl, Hydroxyalkyl oder Alkenyl mit jeweils bis zu 6 Kohlenstoffatomen, Cycloalkyl mit bis zu 6 Kohlenstoffatomen und vorzugsweise 2 bis 5 Kohlenstoffatomen oder Polyoxyalkylen mit bis zu 10 Kohlenstoffatomen,
- $R_3$ und $R_4$, die identisch oder voneinander verschieden sind, Alkyl, Hydroxyalkyl oder Carboxyalkyl mit bis zu 6 Kohlenstoffatomen in jeder Alkylgruppe oder Polyoxyalkylen mit bis zu 10 Kohlenstoffatomen, wobei die Gruppen $R_3$ und $R_4$ im übrigen zusammen mit dem Stickstoffatom, an das sie gebunden sind, einen N-Heterocyclus bilden können,
- n eine ganze Zahl von 2 bis 10.

3. Verwendung nach einem der Ansprüche 1 oder 2, dadurch gekennzeichnet, daß das Anion unter den Halogeniden ausgewählt ist.

4. Verwendung nach einem der vorhergehenden Ansprüche, dadurch gekennzeichnet, daß das Anion Chlorid ist.

5. Verwendung nach einem der Ansprüche 1 bis 4, dadurch gekennzeichnet, daß die zweite Verbindung ethoxyliertem Stearamidopropyldiammonium-chlorid-phosphat, ethoxyliertem Linoleamidopropyldiammonium-chlorid-phosphat, ethoxyliertem Cocamidopropyldiammonium-chlorid-phosphat und ethoxyliertem Borageamidopropyldiammonium-chloridphosphat ausgewählt ist.

6. Verwendung nach einem der vorhergehenden Ansprüche, dadurch gekennzeichnet, daß die erste Verbindung unter niederen Alkoholen mit einer Kohlenstoffanzahl von höchstens 6 und den gegebenenfalls ethoxylierten Polyethylenglykolen mit einer Kohlenstoffanzahl von höchstens 50 sowie deren Gemischen ausgewählt ist.

7. Verwendung nach einem der vorhergehenden Ansprüche, dadurch gekennzeichnet, daß die erste Verbindung unter Propylenglykol, Glycerin, Sorbit, Dipropylenglykol, Butylenglykol, Hexylenglykol, Mannit und deren Gemischen ausgewählt ist.

8. Verwendung nach einem der Ansprüche 1 bis 7, dadurch gekennzeichnet, daß die erste Verbindung unter den PEG mit 6 bis 14 Ethylenoxideinheiten ausgewählt ist.

9. Verwendung nach einem der vorhergehenden Ansprüche, dadurch gekennzeichnet, daß die Zusammensetzung ferner mindestens ein Fett ohne Estergruppe enthält.

10. Verwendung nach Anspruch 9, dadurch gekennzeichnet, daß das Fett unter den Siliconwachsen und den synthetischen Ölen ohne Estergruppen ausgewählt ist.

11. Verwendung nach einem der Ansprüche 9 oder 10, dadurch gekennzeichnet, daß das Fett ohne Estergruppen in einem Anteil von höchstens 20 Gew.-%, bezogen auf das Gesamtgewicht der Zusammensetzung, vorliegt.

12. Verwendung nach einem der vorhergehenden Ansprüche, dadurch gekennzeichnet, daß sie ferner mindestens einen Hilfsstoff ohne Estergruppen enthält, der unter den Konservierungsmitteln, Puffern, Parfums, hydrophilen Wirkstoffen und Maskierungsmitteln ausgewählt ist.

13. Verwendung nach einem der vorhergehenden Ansprüche, dadurch gekennzeichnet, daß die erste Verbindung in einem Anteil von höchstens 20 Gew.-%, bezogen auf das Gesamtgewicht der Zusammensetzung, vorliegt.

14. Verwendung nach einem der vorhergehenden Ansprüche, dadurch gekennzeichnet, daß die erste Verbindung, wenn sie ein niederer Alkohol ist, in einem Anteil von 0,5 bis 15 Gew.-%, bezogen auf das Gesamtgewicht der Zusammensetzung, vorliegt.

15. Verwendung nach einem der vorhergehenden Ansprüche, dadurch gekennzeichnet, daß die erste Verbindung, wenn sie ein Polyethylenglykol ist, in einem Anteil von höchstens 15 Gew.-%, bezogen auf das Gesamtgewicht der Zusammensetzung, vorliegt.

16. Verwendung nach einem der vorhergehenden Ansprüche, dadurch gekennzeichnet, daß die erste Verbindung, wenn sie ein Polyethylenglykol ist, in einem Anteil von 5 bis 15 Gew.-%, bezogen auf das Gesamtgewicht der Zusammensetzung, vorliegt.

17. Verwendung nach einem der vorhergehenden Ansprüche, dadurch gekennzeichnet, daß die zweite Verbindung in einem Anteil von 0,5 bis 3 Gew.-%, bezogen auf das Gesamtgewicht der Zusammensetzung, vorliegt.

18. Verwendung nach einem der vorhergehenden Ansprüche, dadurch gekennzeichnet, daß die zweite Verbindung in einem Anteil von 1 bis 2,5 Gew.-%, bezogen auf das Gesamtgewicht der Zusammensetzung, vorliegt.

19. Verfahren zum Abschminken der geschminkten Haut und/oder der geschminkten Augen und/oder der geschminkten Wimpern und/oder der geschminkten Augenbrauen, dadurch gekennzeichnet, daß auf die geschminkte Haut und/oder die geschminkten Augen und/oder die geschminkten Wimpern und/oder die geschminkten Augenbrauen eine Zusammensetzung nach einem der Ansprüche 1 bis 18 aufgetragen wird und daß gegebenenfalls danach gespült wird.